# EUROPEAN PATENT SPECIFICATION

(11) **EP 3 547 857 B1**
(45) Date of publication and mention of the grant of the patent: **09.08.2023**
(21) Application number: 17804583.7
(22) Date of filing: 29.11.2017
(51) Int. Cl.: A24F 40/485, A24F 40/40

(54) **AEROSOL-GENERATING SYSTEM HAVING AN OUTER HOUSING**
AEROSOLERZEUGUNGSSYSTEM MIT AUSSENGEHÄUSE
SYSTÈME DE PRODUCTION D'AÉROSOL AYANT UN BOÎTIER EXTERNE

(30) Priority: 30.11.2016 EP 16201558
(43) Date of publication of application: 09.10.2019
(73) Proprietor: Philip Morris Products S.A., 2000 Neuchâtel (CH)
(72) Inventor: SAYGILI, Ali Murat, 2000 Neuchâtel (CH)
(74) Representative: Dowling, Ian
(86) International application number: PCT/EP2017/080869
(87) International publication number: WO 2018/099999

(56) References cited:
- EP-A1- 2 022 350
- EP-A1- 3 095 338
- WO-A1-2015/177253
- WO-A1-2015/197627
- WO-A1-2017/108721
- WO-A2-2014/187770
- GB-A- 2 502 052
- US-A1- 2016 183 593

## Description

The present invention relates to an aerosol-generating system comprising an aerosol-generating device and a cartridge assembly each comprising an outer housing forming part of a system outer housing.

Known handheld aerosol-generating systems typically comprise an aerosol-generating device comprising a battery, control electronics and an electric heater for heating an aerosol-generating article designed specifically for use with the aerosol-generating device. In some examples, the aerosol-generating article comprises an aerosol-forming substrate, such as a tobacco rod or a tobacco plug, and the heater contained within the aerosol-generating device is inserted into or around the aerosol-forming substrate when the aerosol-generating article is inserted into the aerosol-generating device.

In an alternative aerosol-generating system, the aerosol-generating article may comprise a capsule containing an aerosol-forming substrate, such as loose tobacco.

In another alternative aerosol-generating system, the aerosol-generating article may comprise a cartridge assembly in which the aerosol-forming substrate comprises a nicotine source and an acid source. In use, the nicotine and the acid are reacted with one another in the gas phase to form an aerosol of nicotine salt particles that is inhaled by the user.

In known aerosol-generating systems it may be difficult for a user to combine an aerosol-generating article with an aerosol-generating device, or to remove an aerosol-generating article from an aerosol-generating device. For example, some known aerosol-generating devices comprise a cavity from which it may be difficult for a user to remove an aerosol-generating article.

WO-A-2014/187770 discloses an aerosol generating system comprising an atomizer for coupling with a power supply to form an electronic cigarette, comprising a liquid supply configured for storing tobacco liquid an a connecting component configured for connecting the liquid supply and the power supply. The connecting component comprises a liquid conducting element and a heating element in contact with the liquid conducting element.

It would be desirable to provide an aerosol-generating system that facilitates improved handling of an aerosol-generating article by a user.

According to the present invention there is provided an aerosol-generating system as set out in claim 1.

As used herein, the term "aerosol-forming substrate" is used to describe a substrate capable of releasing volatile compounds, which can form an aerosol. The aerosols generated from aerosol-forming substrates of cartridge assembly assemblies according to the invention may be visible or invisible and may include vapours (for example, fine particles of substances, which are in a gaseous state, that are ordinarily liquid or solid at room temperature) as well as gases and liquid droplets of condensed vapours.

As used herein, the terms "upstream" and "downstream" are used to describe the relative positions of components, or portions of components, of aerosol-generating systems in relation to the direction of air drawn through the aerosol-generating systems during use. During use air is drawn in a downstream direction, from an upstream portion of the aerosol-generating system.

Advantageously, the aerosol-generating system according to the present invention comprises a cartridge assembly having an outer housing that forms part of the system outer housing, which may facilitate grasping of the cartridge assembly by a user.

At least a first part of a downstream edge of a device outer housing abuts at least a first part of an upstream edge of the cartridge assembly outer housing when a portion of the cartridge assembly inner housing is received within the device cavity. Advantageously, this may facilitate correct insertion of the cartridge assembly inner housing into the device cavity by a user. When the first part of the downstream edge of the device outer housing abuts the first part of the upstream edge of the cartridge assembly outer housing, the user knows that the cartridge assembly inner housing has been fully inserted into the device cavity. When the first part of the downstream edge of the device outer housing abuts the first part of the upstream edge of the cartridge assembly outer housing, further insertion of the cartridge assembly inner housing into the device cavity is prevented.

The device cavity is configured to receive only a portion of the cartridge assembly inner housing. The device cavity may be configured to receive substantially the entire cartridge assembly inner housing.

The cartridge assembly may comprise a mouthpiece and a cartridge, wherein the cartridge comprises the aerosol-forming substrate. Advantageously, providing a mouthpiece as part of the cartridge assembly may facilitate hygienic use of the aerosol-generating system. Providing the mouthpiece as part of the cartridge assembly ensures that the mouthpiece is discarded and replaced with a new mouthpiece when the cartridge assembly is discarded and replaced with a new cartridge assembly.

Preferably, the cartridge comprises the cartridge assembly inner housing and the mouthpiece comprises the cartridge assembly outer housing, wherein the cartridge is positioned within the mouthpiece. Advantageously, providing and combining a separate mouthpiece and cartridge to form the cartridge assembly may facilitate manufacture of the cartridge assembly. For example, cartridge can be constructed with a shape that facilitates filling of the cartridge assembly inner housing with the aerosol-forming substrate during manufacture, and the mouthpiece can be constructed with a shape that facilitates cooperation with the device outer housing when the cartridge assembly is combined with the aerosol-generating device.

Preferably, the mouthpiece comprises a mouthpiece air outlet extending through the cartridge assembly outer housing.

The cartridge assembly may have a length of between about 32 millimetres and about 40 millimetres, preferably between about 33 millimetres and about 39 millimetres, more preferably between about 34 millimetres and about 38 millimetres, more preferably between about 35 millimetres and about 37 millimetres.

When the first part of the downstream edge of the device outer housing abuts the first part of the upstream edge of the cartridge assembly outer housing, preferably the overlap between the cartridge assembly and the aerosol-generating device is between about 10 percent and about 100 percent of the length of the cartridge assembly. Preferably, the overlap between the cartridge assembly and the aerosol-generating device is between about 20 percent and about 99 percent, more preferably between about 30 percent and about 98 percent, more preferably between about 40 percent and about 97 percent, more preferably between about 50 percent and about 96 percent, more preferably between about 60 percent and about 95 percent, more preferably between about 70 percent and about 90 percent, more preferably between about 75 percent and about 85 percent of the length of the cartridge assembly.

The upstream edge of the cartridge assembly outer housing may be formed by an upstream edge of the mouthpiece. Substantially all of the cartridge assembly outer housing may form the mouthpiece.

The mouthpiece may be formed by only a first portion of the cartridge assembly outer housing. The mouthpiece may extend downstream from a second portion of the cartridge assembly outer housing, wherein the upstream edge of the cartridge assembly outer housing is formed by an upstream edge of the second portion of the cartridge assembly outer housing.

Preferably, one of the downstream edge of the device outer housing and the upstream edge of the cartridge assembly outer housing comprises a guide slot, wherein the other of the downstream edge of the device outer housing and the upstream edge of the cartridge assembly outer housing comprises a guide projection. The guide slot is configured to receive the guide projection when the at least a portion of the cartridge assembly inner housing is received within the device cavity. Advantageously, the combination of a guide slot and a guide projection may facilitate insertion of the cartridge assembly inner housing into the device cavity in the correct orientation.

The aerosol-generating system may comprise a single guide slot and a single guide projection.

The aerosol-generating system may comprise a plurality of guide slots and a plurality of guide projections, wherein each guide slot is configured to receive one of the guide projections. All of the guide slots may be provided by the device outer housing. All of the guide slots may be provided by the cartridge assembly outer housing. Some of the guide slots may be provided by the device outer housing and some of the guide slots may be provided by the cartridge assembly outer housing. Preferably, the plurality of guide slots and the plurality of guide projections are configured so that each guide projection can be received in a corresponding guide slot only in a single orientation of the cartridge assembly with respect to the aerosol-generating device. The guide slots and the guide projections may be positioned about the outer housings of the aerosol-generating device and the cartridge assembly with a rotational asymmetry.

At least a portion of the device outer housing may overlie the device inner housing so that at least a portion of the downstream edge of the device outer housing is flush with at least a portion of a downstream edge of the device inner housing.

Advantageously, such embodiments may substantially reduce the risk of a user contacting the device inner housing when the cartridge assembly is removed from the aerosol-generating device, which may be desirable if the device inner housing becomes hot during operation of the aerosol-generating system.

Advantageously, such embodiments may minimise the size of the cartridge assembly outer housing, which may minimise the amount of waste when the cartridge assembly is discarded after use.

At least a portion of the cartridge assembly outer housing may overlie at least a portion of the cartridge assembly inner housing to define a cartridge assembly cavity between the cartridge assembly outer housing and the cartridge assembly inner housing. The aerosol-generating system is configured so that at least a portion of the device inner housing is received within the cartridge assembly cavity when the at least a portion of the cartridge assembly inner housing is received within the device cavity.

Advantageously, such embodiments may increase the length of the cartridge assembly outer housing, which may facilitate handling of the cartridge assembly by a user. In embodiments in which part of the cartridge assembly outer housing defines a mouthpiece, increasing the length of the cartridge assembly outer housing may advantageously facilitate handling of the cartridge assembly without touching the mouthpiece.

The aerosol-generating device comprises a device airflow inlet extending through the device inner housing and in fluid communication with the device cavity. The aerosol-generating device may comprise a single device airflow inlet. The aerosol-generating device may comprise a plurality of device airflow inlets.

The aerosol-generating system is configured so that, during use, some of the airflow entering the device cavity through the device airflow inlet bypasses the cartridge assembly, and some of the airflow entering the device cavity through the device airflow inlet flows through the substrate compartment of the cartridge assembly. In such embodiments, the device airflow inlet functions as a combined air inlet.

The device airflow inlet may comprise a first airflow inlet configured for fluid communication with an upstream end of the substrate compartment when the at least a portion of the cartridge assembly inner housing is received within the device cavity, and wherein the device airflow inlet comprises a second airflow inlet configured for fluid communication with a downstream end of the substrate compartment when the at least a portion of the cartridge assembly inner housing is received within the device cavity. The first and second airflow inlets are the same airflow inlet and comprising a combined air inlet.

Preferably, the aerosol-generating system comprises a system airflow inlet extending through the system outer housing. In embodiments in which the aerosol-generating device comprises at least one device airflow inlet, preferably the system airflow inlet is in fluid communication with the at least one device airflow inlet.

The aerosol-generating system may comprise a single system airflow inlet. The aerosol-generating device may comprise a plurality of system airflow inlets.

The system airflow inlet may comprise an aperture extending through the device outer housing. The system airflow inlet may comprise an aperture extending through the cartridge assembly outer housing.

The aerosol-generating system may be configured so that, when the first part of the downstream edge of the device outer housing abuts the first part of the upstream edge of the cartridge assembly outer housing, a second part of the downstream edge of the device outer housing is spaced apart from a second part of the upstream edge of the cartridge assembly outer housing to define the system airflow inlet between the second part of the downstream edge of the device outer housing and the second part of the upstream edge of the cartridge assembly outer housing.

Preferably, the aerosol-generating system comprises a system airflow outlet in fluid communication with at least one of the downstream end of the device cavity and the downstream end of the substrate compartment. In embodiments in which the cartridge assembly outer housing defines a mouthpiece having a mouthpiece air outlet, preferably the mouthpiece air outlet forms a system airflow outlet.

The aerosol-generating system may comprise a single system airflow outlet. The aerosol-generating device may comprise a plurality of system airflow outlets.

The aerosol-forming substrate may comprise a solid aerosol-forming substrate. The aerosol-forming substrate may comprise tobacco. The aerosol-forming substrate may comprise a tobacco-containing material containing volatile tobacco flavour compounds which are released from the substrate upon heating. The aerosol-forming substrate may comprise a non-tobacco material. The aerosol-forming substrate may comprise tobacco-containing material and non-tobacco containing material.

The aerosol-forming substrate may include at least one aerosol-former. Suitable aerosol-formers include, but are not limited to: polyhydric alcohols, such as propylene glycol, triethylene glycol, 1 ,3-butanediol and glycerine; esters of polyhydric alcohols, such as glycerol mono-, di- or triacetate; and aliphatic esters of mono-, di- or polycarboxylic acids, such as dimethyl dodecanedioate and dimethyl tetradecanedioate.

Preferred aerosol formers are polyhydric alcohols or mixtures thereof, such as propylene glycol, triethylene glycol, 1,3-butanediol and, most preferred, glycerine.

The aerosol-forming substrate may comprise a single aerosol former. The aerosol-forming substrate may comprise a combination of two or more aerosol formers.

The aerosol-forming substrate may have an aerosol former content of greater than approximately 5 percent on a dry weight basis.

The aerosol-forming substrate may have an aerosol former content of between approximately 5 percent and approximately 30 percent on a dry weight basis.

The aerosol-forming substrate may have an aerosol former content of approximately 20 percent on a dry weight basis.

The aerosol-forming substrate may comprise a nicotine source positioned within the substrate compartment.

The aerosol-forming substrate may comprise a liquid aerosol-forming substrate. The liquid aerosol-forming substrate may comprise a nicotine solution. The liquid aerosol-forming substrate may comprise a tobacco-containing material comprising volatile tobacco flavour compounds which are released from the liquid upon heating. The liquid aerosol-forming substrate may comprise a non-tobacco material. The liquid aerosol-forming substrate may include water, solvents, ethanol, plant extracts and natural or artificial flavours. The liquid aerosol-forming substrate may further comprise an aerosol former.

Preferably, the liquid aerosol-forming substrate is impregnated into a carrier material positioned within the substrate compartment.

Preferably, the carrier material has a density of between about 0.1 grams/cubic centimetre and about 0.3 grams/cubic centimetre.

Preferably, the carrier material has a porosity of between about 15 percent and about 55 percent.

The carrier material may comprise one or more of glass, cellulose, ceramic, stainless steel, aluminium, polyethylene (PE), polypropylene, polyethylene terephthalate (PET), poly(cyclohexanedimethylene terephthalate) (PCT), polybutylene terephthalate (PBT), polytetrafluoroethylene (PTFE), expanded polytetrafluoroethylene (ePTFE), and BAREX^{®}.

Preferably, the carrier material is chemically inert with respect to the liquid aerosol-forming material.

The substrate compartment may be a single substrate compartment. The aerosol-forming substrate may comprise at least one of a solid aerosol-forming substrate and a liquid aerosol-forming substrate, as described herein.

The substrate compartment may comprise a first substrate compartment and a second substrate compartment, wherein the aerosol-forming substrate comprises a nicotine source positioned within the first substrate compartment and an acid source positioned within the second substrate compartment.

The nicotine source may comprise at least one of nicotine, nicotine base or a nicotine salt.

The nicotine source may comprise a first carrier material impregnated with nicotine. Suitable carrier materials are described herein. The nicotine source may comprise a first carrier material impregnated with between about 1 milligram and about 50 milligrams of nicotine. The nicotine source may comprise a first carrier material impregnated with between about 1 milligram and about 40 milligrams of nicotine. Preferably, the nicotine source comprises a first carrier material impregnated with between about 3 milligrams and about 30 milligrams of nicotine. More preferably, the nicotine source comprises a first carrier material impregnated with between about 6 milligrams and about 20 milligrams of nicotine. Most preferably, the nicotine source comprises a first carrier material impregnated with between about 8 milligrams and about 18 milligrams of nicotine.

In embodiments in which the first carrier material is impregnated with nicotine base or a nicotine salt, the amounts of nicotine recited herein are the amount of nicotine base or amount of ionised nicotine, respectively.

The first carrier material may be impregnated with liquid nicotine or a solution of nicotine in an aqueous or non-aqueous solvent.

The first carrier material may be impregnated with natural nicotine or synthetic nicotine. The acid source may comprise an organic acid or an inorganic acid.

Preferably, the acid source comprises an organic acid, more preferably a carboxylic acid, most preferably an alpha-keto or 2-oxo acid or lactic acid.

The acid source may comprise an acid selected from the group consisting of 3-methyl-2-oxopentanoic acid, pyruvic acid, 2-oxopentanoic acid, 4-methyl-2-oxopentanoic acid, 3-methyl-2-oxobutanoic acid, 2-oxooctanoic acid, lactic acid and combinations thereof. The acid source may comprise pyruvic acid or lactic acid. Preferably, the acid source comprises lactic acid.

The acid source may comprise a second carrier material impregnated with acid. Suitable carrier materials are described herein. The acid source may be a lactic acid source comprising a second carrier material impregnated with between about 2 milligrams and about 60 milligrams of lactic acid. Preferably, the lactic acid source comprises a second carrier material impregnated with between about 5 milligrams and about 50 milligrams of lactic acid. More preferably, the lactic acid source comprises a second carrier material impregnated with between about 8 milligrams and about 40 milligrams of lactic acid. Most preferably, the lactic acid source comprises a second carrier material impregnated with between about 10 milligrams and about 30 milligrams of lactic acid.

The first carrier material and the second carrier material may be the same or different. Advantageously, the first carrier material is chemically inert with respect to nicotine. Advantageously, the second carrier material is chemically inert with respect to the acid.

The cartridge assembly may further comprise a flavourant. Preferably, the flavourant is positioned within the substrate compartment. In embodiments in which the aerosol-forming substrate comprises a liquid impregnated into a carrier material, preferably the flavourant is a liquid flavourant impregnated into the carrier material. Suitable flavourants include, but are not limited to, menthol.

The cartridge assembly inner housing may further define a heating compartment.

The electrical heater may comprise a resistive heating element positioned within the device cavity, wherein the heating compartment is configured to receive the resistive heating element when the at least a portion of the cartridge assembly inner housing is received within the device cavity.

The electrical heater may comprise an inductive heating element, wherein the cartridge assembly further comprises a susceptor positioned within the heating compartment.

Preferably, the aerosol-generating device further comprises an electrical power supply configured to supply electrical power to the electrical heater. The electrical power supply may comprise a direct current (DC) source. In preferred embodiments, the electrical power supply comprises a battery. The electrical power supply may comprise a Nickel-metal hydride battery, a Nickel cadmium battery, or a Lithium based battery, for example a Lithium-Cobalt, a Lithium-Iron-Phosphate or a Lithium-Polymer battery.

Preferably, the aerosol-generating device further comprises a controller configured to control a supply of electrical power from the electrical power supply to the electrical heater.

The device inner housing, the device outer housing, the cartridge assembly inner housing and the cartridge assembly outer housing may be formed from any suitable material or combination of materials. Suitable materials include, but are not limited to, aluminium, polyether ether ketone (PEEK), polyimides, such as Kapton^{®}, polyethylene terephthalate (PET), polyethylene (PE), high-density polyethylene (HDPE), polypropylene (PP), polystyrene (PS), fluorinated ethylene propylene (FEP), polytetrafluoroethylene (PTFE), polyoxymethylene (POM), epoxy resins, polyurethane resins, vinyl resins, liquid crystal polymers (LCP) and modified LCPs, such as LCPs with graphite or glass fibres.

The device inner housing, the device outer housing, the cartridge assembly inner housing and the cartridge assembly outer housing may be formed from the same or different materials.

The cartridge assembly inner housing may be formed from one or more materials that are nicotine-resistant. The cartridge assembly inner housing may be formed from one or more materials that are acid-resistant.

The substrate compartment of the cartridge assembly may be coated with one or more nicotine-resistant materials. In embodiments in which the substrate compartment comprises a first substrate compartment and a second substrate compartment, the first substrate compartment may be coated with one or more nicotine-resistance materials and the second substrate compartment may be coated with one or more acid-resistant materials.

Examples of suitable nicotine-resistant materials and acid-resistant materials include, but are not limited to, polyethylene (PE), polypropylene (PP), polystyrene (PS), fluorinated ethylene propylene (FEP), polytetrafluoroethylene (PTFE), epoxy resins, polyurethane resins, vinyl resins and combinations thereof.

Use of one or more nicotine-resistant materials may advantageously enhance the shelf life of the cartridge assembly.

Use of one or more acid-resistant materials may advantageously enhance the shelf life of the cartridge assembly.

Embodiments of the invention will now be described, by way of example only, with reference to the accompanying drawings, in which:
Figure 1 shows a perspective view of an aerosol-generating system according to a first embodiment of the present invention;
Figure 2 shows the aerosol-generating system of Figure 1 with the cartridge assembly coupled to the aerosol-generating device;
Figure 3 shows a schematic cross-sectional view of the aerosol-generating system of Figure 2;
Figure 4 shows a perspective view of an aerosol-generating system according to a second embodiment of the present invention; and

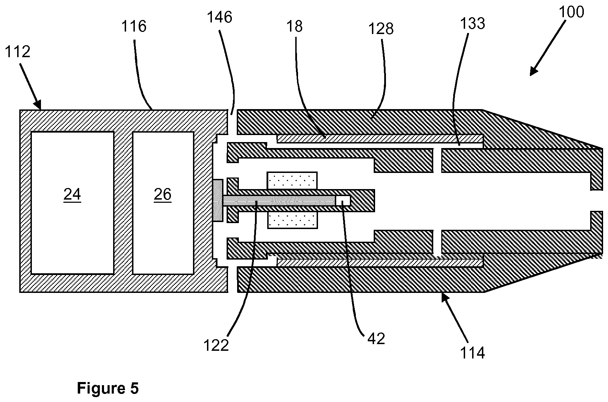

Figure 5 shows a schematic cross-sectional view of the aerosol-generating system of Figure 4.

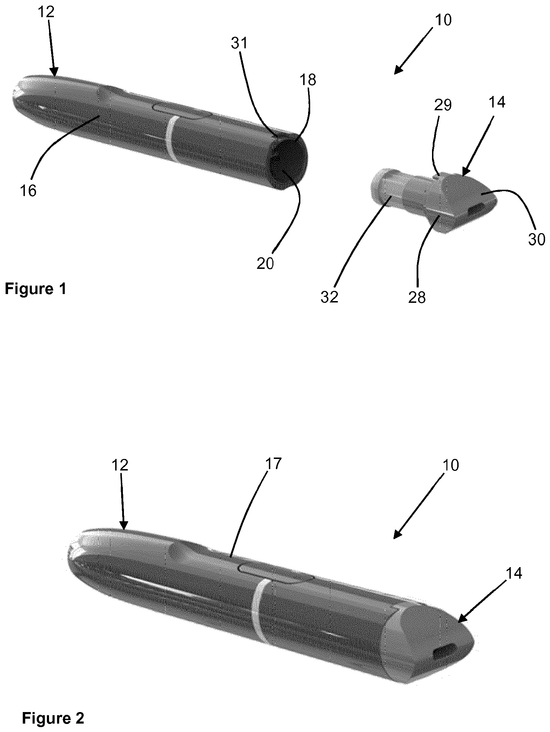

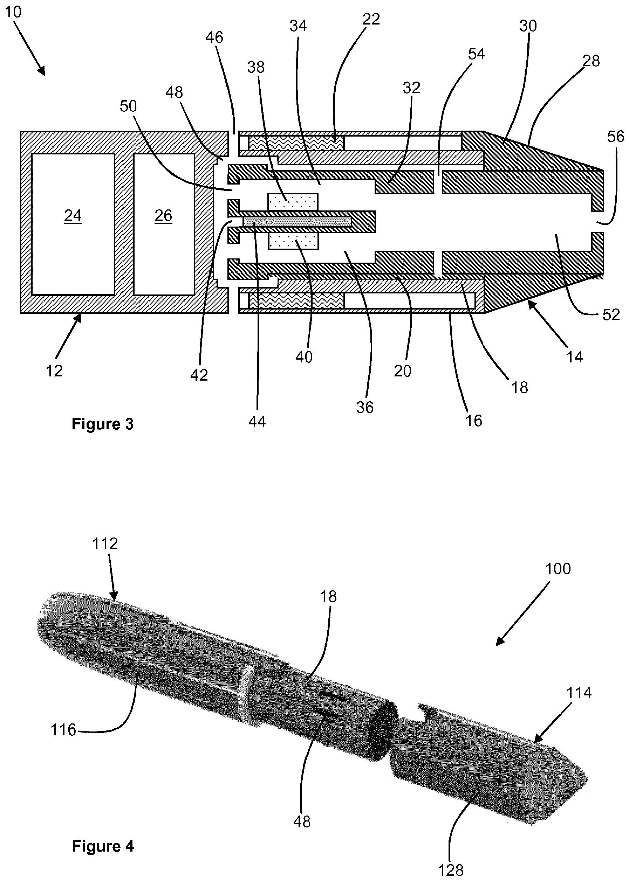

Figure 1 shows an aerosol-generating system 10 according to a first embodiment of the present invention. The aerosol-generating system 10 comprises an aerosol-generating device 12 and a cartridge assembly 14. Figures 2 and 3 show the cartridge assembly 14 coupled to the aerosol-generating device 12.

The aerosol-generating device comprises a device outer housing 16, and a device inner housing 18 that defines a device cavity 20 for receiving a portion of the cartridge assembly 14. As shown in Figure 3, which shows a schematic cross-sectional view of the aerosol-generating system 10, the aerosol-generating device 12 further comprises an electrical heater 22, an electrical power supply 24, and a controller 26 for controlling a supply of electrical power from the electrical power supply 24 to the electrical heater 22. The electrical heater 22 is an annular inductive heater and the electrical power supply 24 is a rechargeable battery.

The cartridge assembly 14 comprises a cartridge assembly outer housing 28 that forms a mouthpiece 30 at a downstream end of the cartridge assembly 14. The cartridge assembly outer housing 28 also forms a guide projection 29 configured for insertion into a guide slot 31 formed by the device outer housing 16 to ensure correct rotational orientation of the cartridge assembly 14 with respect to the aerosol-generating device 12. When the cartridge assembly 14 is coupled to the aerosol-generating device 12 an upstream edge of the cartridge assembly outer housing 28 abuts a downstream edge of the device outer housing 16. The cartridge assembly outer housing 28 and the device outer housing 16 together form a system outer housing 17.

A cartridge assembly inner housing 32 is configured for insertion into the device cavity 20 of the aerosol-generating device 12. The cartridge assembly inner housing 32 defines a first substrate compartment 34 and a second substrate compartment 36, the cartridge assembly 14 further comprising a first aerosol-forming substrate 38 positioned in the first substrate compartment 34 and a second aerosol-forming substrate 40 positioned in the second substrate compartment 36. The first aerosol-forming substrate 38 comprises a nicotine source and the second aerosol-forming substrate 40 comprises an acid source.

The cartridge assembly inner housing 32 also defines a heating compartment 42, the cartridge assembly 14 comprising a susceptor 44 positioned within the heating compartment 42.

During use of the aerosol-generating system 10, the controller 26 controls a supply of electrical power from the electrical power supply 24 to the electrical heater 22 to energize the electrical heater 22. The electrical heater 22 inductively heats the susceptor 44, which heats the first and second aerosol-forming substrates 38, 40.

When a user draws on the downstream end of the aerosol-generating system 10 air is drawn into the aerosol-generating system 10 through system airflow inlets 46 extending through the device outer housing 16. Air entering the aerosol-generating system 10 through the system airflow inlets 46 enters the device cavity 20 via device airflow inlets 48 extending through the device inner housing 18. Each device airflow inlet 48 is a combined air inlet so that air entering through each device airflow inlet 48 is divided into ventilation air and mainstream air.

The mainstream air is directed to the upstream end of the device cavity 20 where it enters the first and second substrate compartments 34, 36 through cartridge assembly airflow inlets 50. As the mainstream air flows through the first and second substrate compartments 34, 36, nicotine vapour and acid vapour from the first and second aerosol-forming substrates 38, 40 are entrained in the mainstream air. The mainstream air containing the nicotine vapour and the acid vapour flows into a mixing chamber 52 at a downstream end of the cartridge assembly 14 where the nicotine vapour and the acid vapour react to form nicotine salt particles.

The ventilation air is directed towards the downstream end of the device cavity 20 where it enters the mixing chamber 52 via ventilation airflow inlets 54 extending through the cartridge assembly inner housing 32. In the mixing chamber 52, the ventilation air mixes with the mainstream air containing the nicotine salt particles to form an aerosol for delivery to the user. The aerosol flows out of the mixing chamber 52 via a mouthpiece airflow outlet 56, which forms a system airflow outlet.

Figure 4 shows an aerosol-generating system 100 according to a second embodiment of the present invention. The construction of the aerosol-generating system 100 is similar to the construction of the aerosol-generating system 10 described with reference to Figures 1 to 3 and like reference numerals are used to designate like parts. Only the differences between the two aerosol-generating systems is described, and the operation of the two aerosol-generating systems is identical.

The aerosol-generating system 100 comprises an aerosol-generating device 112 comprising a device outer housing 116 that is shortened compared to the device outer housing 16 of the aerosol-generating system 10. Therefore, the device outer housing 116 does not overlie the device inner housing 18.

To accommodate the shortened device outer housing 116, the aerosol-generating system 100 comprises a cartridge assembly 114 having an elongated cartridge assembly outer housing 128 that overlies the cartridge assembly inner housing 32 to define a cartridge assembly cavity 133 between the cartridge assembly outer housing 128 and the cartridge assembly inner housing 32. When the cartridge assembly 114 is coupled to the aerosol-generating device 112, the device inner housing 18 is received in the cartridge assembly cavity 133.

In the aerosol-generating system 100, the system airflow inlets 146 are formed between the cartridge assembly outer housing 128 and the device outer housing 116 when the cartridge assembly 114 is coupled to the aerosol-generating device 112. The downstream edge of the device outer housing 116 and the upstream edge of the cartridge assembly outer housing 128 are shaped so that a first part of the upstream edge of the cartridge assembly outer housing 128 abuts a first part of the downstream edge of the device outer housing 116. The downstream edge of the device outer housing 116 and the upstream edge of the cartridge assembly outer housing 128 are shaped so that a second part of the upstream edge of the cartridge assembly outer housing 128 is spaced apart from a second part of the downstream edge of the device outer housing 116 to form the system airflow inlets 146 between the second parts of the upstream edge of the cartridge assembly outer housing 128 and the downstream edge of the device outer housing 116.

The aerosol-generating device 112 comprises an electrical heater 122, the electrical heater 122 comprising a resistive heating element extending into the device cavity 20 at an upstream end of the device cavity 20. The heating compartment 42 of the cartridge assembly 114 is configured to receive the electrical heater 122.

## Claims

1. An aerosol-generating system (10) comprising:
an aerosol-generating device (12) comprising:
a device inner housing (18) defining a device cavity (20);
a device airflow inlet (48) extending through the device inner housing (18) and in fluid communication with the device cavity (20);
an electrical heater (22); and
a device outer housing (16);
a cartridge assembly (14) comprising:
a cartridge assembly inner housing (32) defining a substrate compartment (34);
an aerosol-forming substrate positioned within the substrate compartment (34); and
a cartridge assembly outer housing (28);
wherein a portion of the cartridge assembly inner housing (32) is configured to be received within the device cavity (20);
wherein, when the portion of the cartridge assembly inner housing (32) is received within the device cavity (20), at least a first part of a downstream edge of the device outer housing (16) abuts at least a first part of an upstream edge of the cartridge assembly outer housing (28) so that the cartridge assembly outer housing (28) and the device outer housing (16) form a system outer housing (17); and
wherein each device airflow inlet (48) is a combined air inlet so that air entering through each device airflow inlet (48) is divided into ventilation air and mainstream air, and wherein the device airflow inlet (48) is configured for fluid communication with an upstream end of the substrate compartment (34) and a downstream end of the substrate compartment (34) when the at least a portion of the cartridge assembly inner housing (32) is received within the device cavity (20), the mainstream air being directed to the upstream end of the substrate compartment, and the ventilation air being directed to the downstream end of the substrate compartment.

2. Aerosol-generating system (10) according to claim 1, wherein the cartridge assembly (14) comprises a mouthpiece (30) and a cartridge, the cartridge comprising the aerosol-forming substrate.

3. An aerosol-generating system (10) according to claim 2, wherein the cartridge comprises the cartridge assembly inner housing (32), wherein the mouthpiece (30) comprises the cartridge assembly outer housing (28), and wherein the cartridge is positioned inside the mouthpiece (30).

4. An aerosol-generating system (10) according to claim 1, 2 or 3, wherein, when the first part of the downstream edge of the device outer housing (16) abuts the first part of the upstream edge of the cartridge assembly outer housing (28), an overlap between the cartridge assembly (14) and the aerosol-generating device (12) is between 10 percent and 100 percent of a length of the cartridge assembly (14).

5. An aerosol-generating system (10) according to any preceding claim, wherein one of the downstream edge of the device outer housing (16) and the upstream edge of the cartridge assembly outer housing (28) comprises a guide slot (31), wherein the other of the downstream edge of the device outer housing (16) and the upstream edge of the cartridge assembly outer housing (28) comprises a guide projection (29), and wherein the guide slot (31) is configured to receive the guide projection (29) when the at least a portion of the cartridge assembly inner housing (32) is received within the device cavity (20).

6. An aerosol-generating system (10) according to any preceding claim, wherein at least a portion of the device outer housing (16) overlies the device inner housing (18) so that at least a portion of the downstream edge of the device outer housing (16) is flush with at least a portion of a downstream edge of the device inner housing (18).

7. An aerosol-generating system (10) according to any of claims 1 to 5, wherein at least a portion of the cartridge assembly outer housing (28) overlies at least a portion of the cartridge assembly inner housing (32) to define a cartridge assembly cavity (133) between the cartridge assembly outer housing (28) and the cartridge assembly inner housing (32), and wherein the aerosol-generating system (10) is configured so that at least a portion of the device inner housing (18) is received within the cartridge assembly cavity (133) when the at least a portion of the cartridge assembly inner housing (32) is received within the device cavity (20).

8. An aerosol-generating system (10) according to any preceding claim, wherein, when the first part of the downstream edge of the device outer housing (16) abuts the first part of the upstream edge of the cartridge assembly outer housing (28), a second part of the downstream edge of the device outer housing (16) is spaced apart from a second part of the upstream edge of the cartridge assembly outer housing (28) to define a system airflow inlet (146) between the second part of the downstream edge of the device outer housing (16) and the second part of the upstream edge of the cartridge assembly outer housing (28).

9. An aerosol-generating system (10) according to any preceding claim, wherein the aerosol-forming substrate comprises a nicotine source positioned within the substrate compartment (34).

10. An aerosol-generating system (10) according to claim 9, wherein the substrate compartment (34) comprises a first substrate compartment (34) and a second substrate compartment (36), and wherein the aerosol-forming substrate comprises the nicotine source positioned within the first substrate compartment (34) and an acid source positioned within the second substrate compartment (36).

11. An aerosol-generating system (10) according to any preceding claim, wherein the cartridge assembly inner housing (32) further defines a heating compartment (42).

12. An aerosol-generating system (10) according to claim 11, wherein the electrical heater (22) comprises a resistive heating element positioned within the device cavity (20), and wherein the heating compartment (42) is configured to receive the resistive heating element when the at least a portion of the cartridge assembly inner housing (32) is received within the device cavity (20).

13. An aerosol-generating system (10) according to claim 11, wherein the electrical heater (22) comprises an inductive heating element, and wherein the cartridge assembly (14) further comprises a susceptor (44) positioned within the heating compartment (42).

## Patentansprüche

1. Aerosolerzeugungssystem (10), umfassend:
eine Aerosolerzeugungsvorrichtung (12), aufweisend:
einen Vorrichtungs-Innenmantel (18), der einen Vorrichtungshohlraum (20) definiert;
einen Vorrichtungsluftstromeinlass (48), der sich durch den Vorrichtungs-Innenmantel (18) erstreckt und in Fluidverbindung mit dem Vorrichtungshohlraum (20) steht;
eine elektrische Heizvorrichtung (22); und
einen Vorrichtungs-Außenmantel (16);
eine Patronenanordnung (14), aufweisend:
einen Patronenanordnung-Innenmantel (32), der eine Substratkammer (34) definiert;
ein innerhalb der Substratkammer (34) positioniertes aerosolbildendes Substrat; und
einen Patronenanordnung-Außenmantel (28);
wobei ein Abschnitt des Patronenanordnung-Innenmantels (32) ausgelegt ist, um innerhalb des Vorrichtungshohlraums (20) aufgenommen zu werden;
wobei, wenn der Abschnitt des Patronenanordnung-Innenmantels (32) in dem Vorrichtungshohlraum (20) aufgenommen ist, wenigstens ein erster Teil einer nachgelagerten Kante des Vorrichtungs-Außenmantels (16) an wenigstens einem ersten Teil einer vorgelagerten Kante des Patronenanordnung-Außenmantels (28) anliegt, sodass der Patronenanordnung-Außenmantel (28) und der Vorrichtungs-Außenmantel (16) einen System-Außenmantel (17) bilden; und
wobei jeder Vorrichtungsluftstromeinlass (48) ein kombinierter Lufteinlass ist, sodass die durch jeden Vorrichtungsluftstromeinlass (48) eintretende Luft in Ventilationsluft und Hauptstromluft unterteilt wird, und wobei der Vorrichtungsluftstromeinlass (48) für eine Fluidverbindung mit einem vorgelagerten Ende der Substratkammer (34) und einem nachgelagerten Ende der Substratkammer (34) ausgelegt ist, wenn der wenigstens eine Abschnitt des Patronenanordnung-Innenmantels (32) innerhalb des Vorrichtungshohlraums (20) aufgenommen ist, wobei die Hauptstromluft zu dem vorgelagerten Ende der Substratkammer geleitet wird, und die Ventilationsluft zu dem nachgelagerten Ende der Substratkammer geleitet wird.

2. Aerosolerzeugungssystem (10) nach Anspruch 1, wobei die Patronenanordnung (14) ein Mundstück (30) und eine Patrone umfasst, wobei die Kartusche das aerosolbildende Substrat umfasst.

3. Aerosolerzeugungssystem (10) nach Anspruch 2, wobei die Patrone den Patronenanordnung-Innenmantel (32) umfasst, wobei das Mundstück (30) den Patronenanordnung-Außenmantel (28) umfasst, und wobei die Patrone innerhalb des Mundstücks (30) positioniert ist.

4. Aerosolerzeugungssystem (10) nach Anspruch 1, 2 oder 3, wobei, wenn der erste Teil der nachgelagerten Kante des Vorrichtungs-Außenmantels (16) an dem ersten Teil der vorgelagerten Kante des Patronenanordnung-Außenmantels (28) anliegt, eine Überlappung zwischen der Patronenanordnung (14) und der Aerosolerzeugungsvorrichtung (12) zwischen 10 Prozent und 100 Prozent einer Länge der Patronenanordnung (14) beträgt.

5. Aerosolerzeugungssystem (10) nach einem beliebigen vorhergehenden Anspruch, wobei eine der nachgelagerten Kante des Vorrichtungs-Außenmantels (16) und der vorgelagerten Kante des Patronenanordnung-Außenmantels (28) einen Führungsschlitz (31) aufweist, wobei die andere der nachgelagerten Kante des Vorrichtungs-Außenmantels (16) und der vorgelagerten Kante des Patronenanordnung-Außenmantels (28) einen Führungsvorsprung (29) aufweist, und wobei der Führungsschlitz (31) zum Aufnehmen des Führungsvorsprungs (29) ausgelegt ist, wenn der wenigstens eine Teil des Patronenanordnung-Innenmantels (32) innerhalb des Vorrichtungshohlraums (20) aufgenommen ist.

6. Aerosolerzeugungssystem (10) nach einem beliebigen vorhergehenden Anspruch, wobei wenigstens ein Teil des Vorrichtungs-Außenmantels (16) über dem Vorrichtungs-Innenmantel (18) liegt, sodass wenigstens ein Teil der nachgelagerten Kante des Vorrichtungs-Außenmantels (16) mit wenigstens einem Teil einer nachgelagerten Kante des Vorrichtungs-Innenmantels (18) bündig ist.

7. Aerosolerzeugungssystem (10) nach einem der Ansprüche 1 bis 5, wobei wenigstens ein Teil des Patronenanordnung-Außenmantels (28) über wenigstens einem Teil des Patronenanordnung-Innenmantels (32) liegt, um einen Patronenanordnungshohlraum (133) zwischen dem Patronenanordnung-Außenmantel (28) und dem Patronenanordnung-Innenmantel (32) zu definieren, und wobei das Aerosolerzeugungssystem (10) derart ausgelegt ist, dass wenigstens ein Teil des Vorrichtungs-Innenmantels (18) innerhalb des Patronenanordnungshohlraums (133) aufgenommen ist, wenn der wenigstens eine Teil des Patronenanordnung-Innenmantels (32) innerhalb des Vorrichtungshohlraums (20) aufgenommen ist.

8. Aerosolerzeugungssystem (10) nach einem beliebigen vorhergehenden Anspruch, wobei, wenn der erste Teil der nachgelagerten Kante des Vorrichtungs-Außenmantels (16) an dem ersten Teil der vorgelagerten Kante des Patronenanordnungs-Außenmantels (28) anliegt, ein zweiter Teil der nachgelagerten Kante des Vorrichtungs-Außenmantels (16) von einem zweiten Teil der nachgelagerten Kante des Patronenanordnungs-Außenmantels (28) beabstandet ist, um einen Systemluftstromeinlass (146) zwischen dem zweiten Teil der nachgelagerten Kante des Vorrichtungs-Außenmantels (16) und dem zweiten Teil der vorgelagerten Kante des Patronenanordnungs-Außenmantels (28) zu definieren.

9. Aerosolerzeugungssystem (10) nach einem beliebigen vorhergehenden Anspruch, wobei das aerosolerzeugende Substrat eine innerhalb der Substratkammer (34) positionierte Nikotinquelle aufweist.

10. Aerosolerzeugungssystem (10) nach Anspruch 9, wobei die Substratkammer (34) eine erste Substratkammer (34) und eine zweite Substratkammer (36) aufweist und wobei das aerosolerzeugende Substrat die in der ersten Substratkammer (34) positionierte Nikotinquelle und eine in der zweiten Substratkammer (36) positionierte Säurequelle umfasst.

11. Aerosolerzeugungssystem (10) nach einem beliebigen vorhergehenden Anspruch, wobei der Patronenanordnung-Innenmantel (32) ferner eine Erwärmungskammer (42) definiert.

12. Aerosolerzeugungssystem (10) nach Anspruch 11, wobei die elektrische Heizvorrichtung (22) ein innerhalb des Vorrichtungshohlraums (20) positioniertes Widerstandsheizelement aufweist und wobei die Erwärmungskammer (42) zum Aufnehmen des Widerstandsheizelements ausgelegt ist, wenn der wenigstens eine Teil des Patronenanordnung-Innenmantels (32) innerhalb des Vorrichtungshohlraums (20) aufgenommen ist.

13. Aerosolerzeugungssystem (10) nach Anspruch 11, wobei die elektrische Heizvorrichtung (22) ein induktives Heizelement aufweist, und wobei die Patronenanordnung (14) ferner einen innerhalb der Erwärmungskammer (42) positionierten Suszeptor (44) aufweist.

## Revendications

1. Système de génération d'aérosol (10) comprenant :
un dispositif de génération d'aérosol (12) comprenant :
un logement intérieur de dispositif (18) définissant une cavité de dispositif (20) ;
une entrée d'écoulement d'air de dispositif (48) s'étendant à travers le logement intérieur de dispositif (18) et en communication fluidique avec la cavité de dispositif (20) ;
un dispositif de chauffage électrique (22) ; et
un logement extérieur de dispositif (16) ;
un ensemble de cartouche (14) comprenant :
un logement intérieur d'ensemble de cartouche (32) définissant un compartiment de substrat (34) ;
un substrat formant aérosol positionné au sein du compartiment de substrat (34) ; et
un logement extérieur d'ensemble de cartouche (28) ;
dans lequel une portion du logement intérieur d'ensemble de cartouche (32) est configurée pour être reçue au sein de la cavité de dispositif (20) ;
dans lequel, lorsque la portion du logement intérieur d'ensemble de cartouche (32) est reçue au sein de la cavité de dispositif (20), au moins une première partie d'un bord aval du logement extérieur de dispositif (16) bute contre au moins une première partie d'un bord amont du logement extérieur d'ensemble de cartouche (28) de sorte que le logement extérieur d'ensemble de cartouche (28) et le logement extérieur de dispositif (16) forment un logement extérieur de système (17) ; et
dans lequel chaque entrée d'écoulement d'air de dispositif (48) est une entrée d'air combinée de sorte que l'air entrant à travers chaque entrée d'écoulement d'air de dispositif (48) est divisé en air de ventilation et air d'écoulement principal, et dans lequel l'entrée d'écoulement d'air de dispositif (48) est configurée pour une communication fluidique avec une extrémité amont du compartiment de substrat (34) et une extrémité aval du compartiment de substrat (34) lorsque l'au moins une portion du logement intérieur d'ensemble de cartouche (32) est reçue au sein de la cavité de dispositif (20), l'air d'écoulement principal étant dirigé vers l'extrémité amont du compartiment de substrat, et l'air de ventilation étant dirigé vers l'extrémité aval du compartiment de substrat.

2. Système de génération d'aérosol (10) selon la revendication 1, dans lequel l'ensemble de cartouche (14) comprend un embout buccal (30) et une cartouche, la cartouche comprenant le substrat formant aérosol.

3. Système de génération d'aérosol (10) selon la revendication 2, dans lequel la cartouche comprend le logement intérieur d'ensemble de cartouche (32), dans lequel l'embout buccal (30) comprend le logement extérieur d'ensemble de cartouche (28), et dans lequel la cartouche est positionnée au sein de l'embout buccal (30).

4. Système de génération d'aérosol (10) selon la revendication 1, 2 ou 3, dans lequel, lorsque la première partie du bord aval du logement extérieur de dispositif (16) bute contre la première partie du bord amont du logement extérieur d'ensemble de cartouche (28), un chevauchement entre l'ensemble de cartouche (14) et le dispositif de génération d'aérosol (12) est entre 10 pour cent et 100 pour cent d'une longueur de l'ensemble de cartouche (14).

5. Système de génération d'aérosol (10) selon l'une quelconque des revendications précédentes, dans lequel l'un du bord aval du logement extérieur de dispositif (16) et du bord amont du logement extérieur d'ensemble de cartouche (28) comprend une fente de guidage (31), dans lequel l'autre du bord aval du logement extérieur de dispositif (16) et du bord amont du logement extérieur d'ensemble de cartouche (28) comprend une saillie de guidage (29), et dans lequel la fente de guidage (31) est configurée pour recevoir la saillie de guidage (29) lorsque l'au moins une portion du logement intérieur d'ensemble de cartouche (32) est reçue au sein de la cavité de dispositif (20).

6. Système de génération d'aérosol (10) selon l'une quelconque des revendications précédentes, dans lequel au moins une portion du logement extérieur de dispositif (16) recouvre le logement intérieur de dispositif (18) de sorte qu'au moins une portion du bord aval du logement extérieur de dispositif (16) est alignée avec au moins une portion d'un bord aval du logement intérieur de dispositif (18).

7. Système de génération d'aérosol (10) selon l'une quelconque des revendications 1 à 5, dans lequel au moins une portion du logement extérieur d'ensemble de cartouche (28) recouvre au moins une portion du logement intérieur d'ensemble de cartouche (32) pour définir une cavité d'ensemble de cartouche (133) entre le logement extérieur d'ensemble de cartouche (28) et le logement intérieur d'ensemble de cartouche (32), et dans lequel le système de génération d'aérosol (10) est configuré de sorte qu'au moins une portion du logement intérieur de dispositif (18) est reçue au sein de la cavité d'ensemble de cartouche (133) lorsque l'au moins une portion du logement intérieur d'ensemble de cartouche (32) est reçue au sein de la cavité de dispositif (20).

8. Système de génération d'aérosol (10) selon l'une quelconque des revendications précédentes, dans lequel, lorsque la première partie du bord aval du logement extérieur de dispositif (16) bute contre la première partie du bord amont du logement extérieur d'ensemble de cartouche (28), une deuxième partie du bord aval du logement extérieur de dispositif (16) est espacée d'une deuxième partie du bord amont du logement extérieur d'ensemble de cartouche (28) pour définir une entrée d'écoulement d'air de système (146) entre la deuxième partie du bord aval du logement extérieur de dispositif (16) et la deuxième partie du bord amont du logement extérieur d'ensemble de cartouche (28).

9. Système de génération d'aérosol (10) selon l'une quelconque des revendications précédentes, dans lequel le substrat formant aérosol comprend une source de nicotine positionnée au sein du compartiment de substrat (34).

10. Système de génération d'aérosol (10) selon la revendication 9, dans lequel le compartiment de substrat (34) comprend un premier compartiment de substrat (34) et un deuxième compartiment de substrat (36), et dans lequel le substrat formant aérosol comprend la source de nicotine positionnée au sein du premier compartiment de substrat (34) et une source d'acide positionnée au sein du deuxième compartiment de substrat (36).

11. Système de génération d'aérosol (10) selon l'une quelconque des revendications précédentes, dans lequel le logement intérieur d'ensemble de cartouche (32) définit en outre un compartiment de chauffage (42).

12. Système de génération d'aérosol (10) selon la revendication 11, dans lequel le dispositif de chauffage électrique (22) comprend un élément de chauffage par résistance positionné au sein de la cavité de dispositif (20), et dans lequel le compartiment de chauffage (42) est configuré pour recevoir l'élément de chauffage par résistance lorsque l'au moins une portion du logement intérieur d'ensemble de cartouche (32) est reçue au sein de la cavité de dispositif (20).

13. Système de génération d'aérosol (10) selon la revendication 11, dans lequel le dispositif de chauffage électrique (22) comprend un élément de chauffage par induction, et dans lequel l'ensemble de cartouche (14) comprend en outre un suscepteur (44) positionné au sein du compartiment de chauffage (42) .
